# EUROPEAN PATENT APPLICATION

(11) **EP 4 556 566 A1**
(43) Date of publication of application: **21.05.2025**
(21) Application number: 23210906.6
(22) Date of filing: 20.11.2023
(51) Int. Cl.: C12N 15/74

(54) **METHOD FOR PRODUCING CURDLAN AND USAGE THEREOF**

(71) Applicant: Justus-Liebig-Universität Gießen, 35390 Gießen (DE)
(72) Inventor: McIntosh, Matthew, 35398 Gießen (DE)
(74) Representative: Stumpf, Peter

(57) **Abstract**

A transcription overriding cassette (TORC) composed for the expression of at least one gene of interest, characterised in that the TORC comprises two parts, one being a first DNA sequence comprising a strong constitutive promoter DNA sequence having at least 50% nucleotide identity with the promoter DNA sequence according to SEQ ID 6 and one being a second DNA sequence having at least 50% nucleotide identity with SEQ ID 23. The first DNA sequence is located directly before the second DNA sequence within the TORC and the first and second DNA sequences are connected directly to each other within the complete sequence of the TORC or are separated from each other within the complete sequence of the TORC by DNA sequences of DNA restriction cleavage sites .

## Description

The invention relates to the production of Curdlan by way of fermentation, especially the optimization of yield by way of genetic modification, and the usage of curdlan, produced this way.

### Description

Curdlan is a water-insoluble polymer that has structure and gelling properties useful in a wide variety of applications such as in medicine, cosmetics, packaging and the food and building industries. The capacity to produce curdlan has been detected in certain soil-dwelling bacteria of various phyla, although the role of curdlan in their survival remains unclear. One of the major limitations of the extensive use of curdlan in industry is the high cost of production during fermentation, partly because production involves specific nutritional requirements such as nitrogen limitation. Engineering of the industrially relevant curdlan-producing strain Agrobacterium sp. ATTC31749 is a promising approach that could decrease the cost of production.

### Field of the invention

Many bacteria produce extracellular polysaccharides (EPS) that are involved in attachment. EPS enable cell-cell attachment, facilitating the formation of flocs (rafts of aggregated cells in aqueous environments) and biofilms through enhancing attachment to surfaces in soils or on plants and animals. In pathogenesis they may confer resistance to host defences, thereby promoting intracellular survival. In non-pathogenic contexts, they may increase access to nutrients and protect against environmental stresses (e.g., desiccation and toxic agents). Some EPS are hydrophilic or gel forming polymers and are loosely adherent to the bacterial surface forming a slime layer (e.g., xanthan from *Xanthomonas campestris* and succinoglycan from *Rhizobia* and *Agrobacteria* species). Others form a discrete capsule, which overlies the cell surface but is not attached to it (e.g., hyaluronan from *Streptococcus* species and *Pasteurella multocida*)*.* In *Azotobacter vinelandii* a calcium alginate gel forms a coat around the resting cyst stage. Some bacteria are known to produce several different EPSs simultaneously, such as *Agrobacterium tumefaciens,* which can produce at least five.

A few EPS are water-insoluble, notably the (1-4)-β-glucan, cellulose, which is produced by some species recoverable from soil, e.g., *Gluconacetobacter xylinus, Agrobacterium tumefaciens,* species of Pseudomonas, Achromobacter, Alcaligenes, Rhizobium, Azotobacter, members of the Enterobacteriaceae, and as a sheath around filamentous cyanobacteria.

Curdlan, a linear (1→3)-β-D-glucan, is another example of a water insoluble EPS. This polysaccharide is produced by various *Agrobacterium* species, some Rhizobium isolates, the Gram-positive *Cellulomonas flavigena* and an isolate of *Rahnella variigena,* a member of the Enterobacteriaceae family. In all these cases cited above from current state of the art, curdlan production in the laboratory and on industrial scope inevitably requires N-limited culture medium with ample carbohydrate, which fits with the general observation that the production of EPS by bacteria is induced by nutritional stress e.g., N, P or S limitation. It is nonetheless remarkable that the capacity for curdlan production is not restricted to a single Class or even a Phylum (e.g., Gram negative), but rather to a handful of bacteria that seem related only by their soil habitat. So far, curdlan production has only been detected in rhizosphere-dwelling bacteria, and is in every case regulated by N-limitation.

The biological role of curdlan has not been demonstrated, but it has been suggested to help form a biofilm that protects against predation, starvation and desiccation. Such a role is consistent with microscopic observations of *C. flavigena,* a soil-dwelling, curdlan-producing bacterium that colonizes and decomposes plant material using cellulose as a source of carbohydrate. However, little information is available on the form of nascent curdlan, its deposition, and the ongoing nature of its interaction with the cell surface. Curdlan is occasionally referred to in the literature as a capsule, although clear evidence for a capsular form has not been provided. Curdlan-producing Agrobacterium strains grown on glucose-rich yeast extract agar medium produce a coherent, aniline blue-staining, pellicle which may be stripped from areas of confluent bacterial growth, giving the appearance of curdlan deposition as a disorganised mass at the colony surface. However, such an appearance does not rule out the possibility of a capsular form. One possibility is that curdlan is produced as a discrete capsule by some cells located strictly in the periphery of a biofilm. This scenario takes into account the strong dependency of curdlan production on oxygen availability. Peripheral cells would in this way have the role of providing curdlan as a protecting layer for the whole population. Alternatively, it is possible that upon production, curdlan helps to form a biofilm which engulfs the colony and forms a nebulous matrix of a biofilm without any discrete individual cell encapsulation. Without being bound to a certain theory, only the former scenario would justify the formal designation of curdlan as a capsule according to the literature.

A single molecule of curdlan can be quite large, consisting of up to 12 000 polymerized glucose residues. Its size, as well as the unique, unbranched (1→3)-β-D-glucan structure confers upon curdlan its unique gelling and high water-retention properties. There have been many reviews of the large body of literature covering the chemistry and physico-chemistry of curdlan, its pharmacological and immunomodulation activities and its commercial applications. Curdlan is widely used, for example, as an additive to cement (super-workable concrete) for the construction industry, an additive to tablet formulations in the pharmaceutical industry, as well as an antiviral agent in immunotherapy and numerous applications in tissue engineering, drug delivery, and other biomedical applications. Moreover, curdlan is one of the most desirable edible polysaccharides because it lacks any unpleasant taste and because of its versatile applications as a thickener and healthy fiber in the food industry. Unfortunately, the industrial use of curdlan is limited by its high cost of production.

The genetic basis of curdlan production has been investigated using Agrobacterium sp. strain ATCC31749. Earlier studies found that the essential genes include a biosynthetic gene cluster, *crdASC,* and *crdR,* which codes for a transcription regulator. CrdS shows some similarity to beta-glycoxyl transferases with repetitive action patterns. The extremely hydrophobic CrdS protein is thought to form a multimeric (possibly an octamer) complex located in the inner membrane. This location is stabilized by seven transmembrane helices and a central large hydrophilic cytoplasmic region which contains the substrate-binding and catalytic motif.

The *crdASC* genes form a gene cluster (i.e., all transcribed in the same direction) within 5 kb of the linear chromosome of Agrobacterium. Upstream of the first gene (*crdA*) in the *crd* cluster is a strong promoter, referred to as *crdP* or Pcrd. It is not known whether Pcrd is the only promoter for the *crdASC* cluster. Transposon insertions into any of the genes *crdA, crdS,* or *crdC* result in the inability to produce curdlan. Loss of curdlan production by the insertion disruption of *crdA* is interesting because of the implication that either *crdA* is essential for curdlan production and/or because of the polar effect of disruption of transcription of the downstream gene *crdS.* The functions of the protein products of the genes *crdA* and *crdC* are not known.

The gene *pssAG,* coding for a phosphatidylserine synthase, also affects curdlan yield. It is likely that specific phospholipids are important for the activity of the synthase complex in the inner membrane of this Gram-negative bacterium. In addition, according to current state of the art curdlan production is strictly governed by complex gene expression regulation which serves to restrict and suppress curdlan production under typical growth conditions. Under such conditions, the genes required for curdlan production are not expressed and no curdlan is produced. Upon encountering the environmental triggers for curdlan production (e.g., low nitrogen and abundant sugars and oxygen) the transcription of the *crdASC* genes increases by almost 100-fold, and this depends upon phosphate accumulation and the stringent response. Considering that curdlan is a polymer of glucose that is destined for the cell exterior, it is reasonable that the expression of genes that incorporates this high-energy molecule into an exopolysaccharide are highly regulated. So far, the only regulatory mechanisms governing curdlan production that have been reported operate at the level of transcription control. This is in contrast to another exopolysaccharide produced by Agrobacterium, succinoglycan, which does not appear to be regulated at the level of transcription.

Curdlan has been intensively studied to increase production and lower costs during fermentation. Two of the major considerations for improving production and lowering the costs are the complex native regulation controlling production and the specific nutritional conditions required during fermentation. These two considerations are related, since the specific conditions required during fermentation drive up the cost of production. Generally, the native regulation serves to restrain curdlan production until certain specific conditions are met. These include low nitrogen, high carbon sources, generous phosphate levels, and high aeration. Some strains of Agrobacterium, e.g., the well-known plant pathogen *Agrobacterium tumefaciens* C58 that is used in plant genetic manipulations, do not produce detectable levels of curdlan, even though they carry the necessary genes (*crdASC* and *crdR*). Other strains, such as Agrobacterium sp. strain ATCC31749, appear to have accumulated one or more spontaneous mutations that have partially relaxed the regulatory control over curdlan production, leading to higher yields. However, even these higher curdlan yielding strains still contain some native regulation that restricts curdlan production, and until now, it was not possible within the state of the art to override these native regulations. For example, according to the current state of the art, curdlan production begins after 72 hours of fermentation. Innovations that remove or decrease this delay in curdlan production could ideally save not only time but also costs associated with longer incubations times (e.g., heating and stirring).

Various genetic modifications of strain ATCC31749 have generally resulted in not more than 10-50% improvement. One possible explanation is that a greater production rate of curdlan is not possible, i.e., that the production rate is limited by cellular metabolism capacities, uptake of carbon source, or lifetime of the bacterial cell. Therefore, according to previous state of the art, no amount of genetic engineering could greatly improve the yield nor does the previous state of the art provide any hint to overcoming these natural restrictions. However, a possibility, revealed for the first time herein, is that a constitutive transcription of the *crdASC* genes is able to yield higher levels of curdlan - an approach that has not been attempted before within the previous state of the art. To state the technical effect, exerted by the invention revealed herein, succinctly: If the native promoter Pcrd that controls the expression of the entire *crdASC* gene cluster is to be replaced with a stronger, unregulated promoter, this leads to a higher curdlan yield. This result is because of the specific situation that curdlan production is highly dependent upon transcription control. Thus, instead of the modification leading to the death of the cell, which might have been expected to happen according to previous state of the art heretofore, the cell responds by producing more curdlan. Related to the technical effect that increased transcription of the *crdASC* genes has on increasing production is the technical effect that a stronger synthetic promoter relaxes the requirement for specific conditions (e.g., high oxygen, low nitrogen) and supporting high curdlan production independently of the nutritional conditions. A third technical effect exerted by the invention is the forcing of cells to produce curdlan without the standard three-day delay. According to the previous state of the art, the fermentation starts with cells in a growth phase that last about three days. Thereafter, the culture enters a second phase in which cell growth has ceased, possibly because of limiting nitrogen. Curdlan production is the hallmark of this second phase. The invention here forces the cells to produce curdlan in both the growth phase and the second phase. This has the effect of increasing overall curdlan yield within a population performing fermentation, as well as shortening the fermentation times necessary for maximum curdlan yield.

The key to engineering a higher transcription rate for the *crdASC* gene cluster is the knowledge of suitable promoters capable of strong, constitutive transcription in Agrobacterium. During investigations on transcription regulation in the Alphaproteobacteria, a strong promoter named promoter PphaP (promoter of gene *phaP*) from the Alphaproteobacterium *Rhodobacter sphaeroides* has been found. It was decided to test whether curdlan production could be improved using PphaP in the industrially used strain Agrobacterium sp. ATTC31749 and the 'wild type' *Agrobacterium tumefaciens* strain C58. DNA sequence alignments using blastn from NCBI on the *crdASC* gene clusters from each of the strains reveals 98.72% nucleotide identity. This analysis is based on 5.3 kb which includes the *crdASC* genes and the 300 bp promoter region, using C58 (in NCBI: Agrobacterium fabrum str. C58) and 31749 (in NCBI: Agrobacterium sp. CGMCC 11546). The key outcome of this comparison is that both 31749 and C58 carry a *crdASC* gene cluster. (However, C58 does not produce quantifiable curdlan levels under standard laboratory conditions.) Therefore, it was investigated if the insertion of PphaP into the chromosome of each Agrobacterium, replacing the native *crdASC* promoter (Pcrd) and driving the expression of the *crdASC* gene cluster, could increase curdlan production in each strain.

### Background of the technology

One way to reduce the cost of production is to develop higher curdlan-yielding strains through genetic engineering (the task of the invention). A previous invention called ACIT involves the insertion of synthetic promoters into a bacterial chromosome to control gene expression. This current invention for improving curdlan production contains some overlap with ACIT. However, this overlap is confined to the use of standard laboratory tools such as the chromosome integrating plasmid (suicide vectors), and standard laboratory proceedures such as conjugation and selection using antibiotic resistance, and are therefore do not represent innovations. In contrast, the innovations in both technologies are non-overlapping. For example, ACIT relies on modified (synthetic promoters) for the control over gene expression, whereas the current invention uses the unmodified promoter PphaP from the bacterium *Rhodobacter sphaeroides.* A second difference is that the ACIT technology involves the use of genes coding for transcription regulators, transcription terminators and inducers for the control of gene expression, whereas this current invention aims for a minimalistic genetic modification, and does not necessarily require any additional genetic elements. The two components necessary are the unmodified PphaP promoter and any standard suicide vector. Nevertheless, additional genetic elements may be present in the cassette revealed herein without reducing the efficacy of expression, so that additional genetic elements are comprised by the scope of the invention. A third major difference is that ACIT, as its name implies (Aphaproteobacteria chromosomally integrating transcription-control cassette), relies on the use of chemical inducer molecules to control expression of target genes, whereas the current invention uses a strong, constitutively active promoter (e. g. PphaP) which actively promotes transcription under all growth conditions and over-rides the activity of the native promoter. In this way, the current invention does not rely on fine-tuning control over gene expression, but is designed for obtaining maximal gene expression of the *crdASC* genes encoding the curdlan biosynthetic complex using the simplest genetic modification possible. Hence, the current invention differs from ACIT because it provides no control over gene expression, but rather an overriding maximal gene expression by the employment of the super-strong promoter, *phaP.* Aside from these major differences in the two technologies, other differences exist. For example, the DNA sequences are almost completely different between the two technologies (with the exception of the suicide vector). The location of insertion of the suicide plasmid into the chromosome is also different. In the case of ACIT, the location of insertion is at any gene of interest, whereas in this current invention, the location of insertion is specifically within the first 500 bp of the *crdA* gene of the *crdASC* gene cluster coding for curdlan production. Thus, the current invention is very different from the ACIT invention and could not have been conceived based on the ACIT innovation.

### Content of the invention

An objective of the present invention is to provide a method for improving the fermentative production of curdlan. In order to achieve the above objective, the present invention provides a new type of transcription overriding cassette which allows modification of the Alphaproteobacterium, especially *Agrobacterium tumefaciens,* in such a way that production of curdlan by the modified bacteria is no longer restricted by availability of nitrogen for bacterial growth. This saves fermentation time and cost for fermentation substrates. The findings revealed herein also provide a feasible way for easily gathering the curdlan from the fermentation broth.

The terms "comprise", "contain", "have" and "include" as used herein can be used synonymously and shall be understood as an open definition, allowing further members or parts or elements or SEQ-ID's. "Consisting" is considered as a closest definition without further elements of the consisting definition feature. Thus "comprising" is broader and contains the "consisting" definition. The term "about" as used herein refers to the same value or a value differing by +/-5 % of the given value. A person of ordinary skill will recognize that additional ranges of concentrations, particle sizes, temperatures etc. within the explicit ranges revealed herein are contemplated and are within the present disclosure.

During investigations on the regulation and deposition of curdlan, it was found that only a part of the bacterial population produced a curdlan capsule. This heterogeneous distribution appeared to be due to the activity of Pcrd, the native promoter responsible for the expression of the *crdASC* biosynthetic gene cluster. To improve curdlan production, Pcrd is replaced by a promoter (PphaP) from another *Alphaproteobacterium, Rhodobacter sphaeroides.* It is found that, compared to Pcrd, PphaP is stronger, homogeneously active, and only mildly affected by nitrogen levels. Consequently, PhaP dramatically boosts (enhances) *crdASC* gene expression and curdlan production. Importantly, the genetic modification according to the invention over-rides the strict nitrogen depletion regulation that presents a hindrance for maximal curdlan production and from nitrogen rich, complex media, demonstrating excellent commercial potential for achieving high yields using cheap substrates under relaxed fermentation conditions.

**Microscopical analysis of curdlan producing Agrobacterium cells reveals curdlan capsule.** The strain ATCC31749 routinely produces curdlan when grown in liquid minimal salts with limiting nitrogen for longer than 3 days, whereas a knockout (KO) strain of *crdA* produces no detectable curdlan (<0.01 g/L). This 3-day delay in curdlan production fits with the knowledge that curdlan producing Agrobacterium cultures undergo a two-stage development: an initial stage involving cell growth, and a later stage capable of producing curdlan provided that nitrogen is limiting and a suitable carbon source is present. The 3-day growth period is also necessary for the appearance of flocs of variable size which are visible to the naked eye in cultures of the curdlan producing strain, but not in those of the *crdA* mutant. From this observation, it may be suspected - without being bound to a certain theory - that curdlan production is associated with floc formation. Detecting floc-formation within the fermenter can therefore be used for controlling the fermentation-process (e.g., setting certain parameters or detecting time for harvesting) in order to optimise curdlan-production without leaving the scope of the invention.

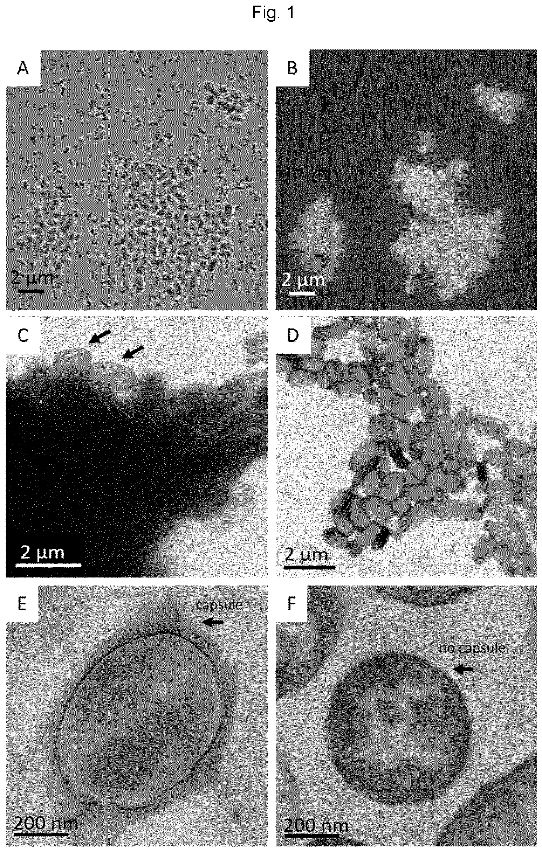

Exemplarily, the cells from a 7-day culture are examined using phase contrast microscopy. Two prominent morphological forms of cells are observable in the curdlan producing cultures. These are a thicker rod form averaging 1.5 × 3.0 µm, located in flocs (cell aggregates) and a slender rod form, averaging 0.5 x 2.0 µm that was planktonic (Fig. 1A). Only the slender rod form is observed in cultures of the *crdA* mutant. When stained with the (1→3)-β-glucan-specific aniline blue fluorochrome (ABF) only the larger form exhibits strong yellow/green fluorescence in a defined zone around the cell periphery (Fig. 1B, showing the same sample after staining). These observations suggest - without being bound by a certain theory - that curdlan is deposited as a capsule and that production of the capsule is restricted to cells located in flocs.

**Electron microscopy of curdlan capsules.** The physical form of native curdlan on the Agrobacterium cell surface is investigated by TEM of uranyl acetate-stained preparations. At low magnification (9600 x), flocs from the curdlan producing culture are heavily stained, as might be expected for encapsulated cells. Occasional cells appear to contain incomplete capsules (Fig. 1C, black arrows). In contrast, the aggregates of cells from the *crdS* mutant culture are quite difficult to find (being very rare) and not heavily stained (Fig. 1D). The fibrillary nature of the capsular material is evident in sections of embedded curdlan producing cells stained with lead citrate-uranyl acetate (Fig. 1E) when compared with the naked surface of the *crdS* mutant cells (Fig. 1F). The use of electron microscopy (Fig. 1C-F) confirms the deposition of curdlan as a capsule - defined as a polysaccharide layer that lies outside the cell envelope. In contrast, bacteria incapable of producing curdlan have a 'clean' surface. These analyses confirm curdlan as a discreet capsule found only on the surface of curdlan producing cells.

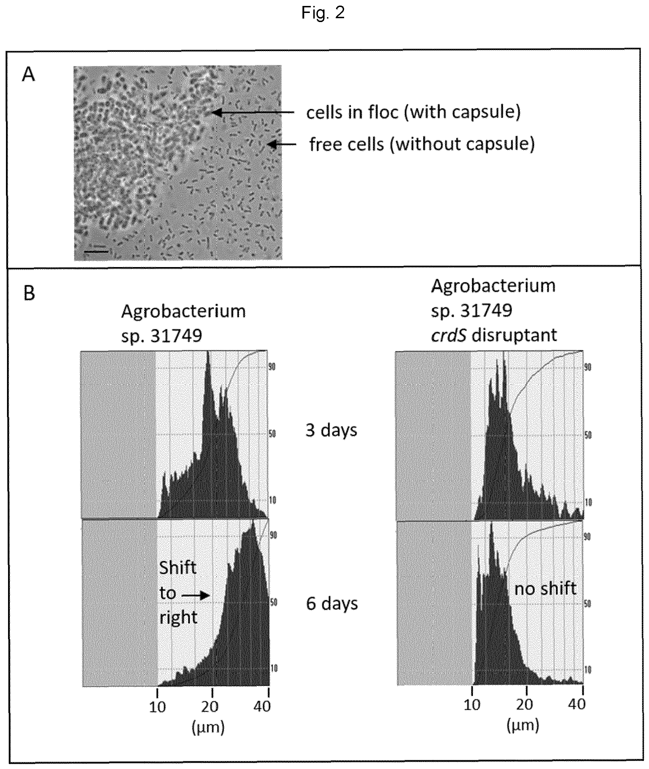

**Formation and organization of flocs.** As noted above, the curdlan producing form is rarely planktonic. Rather, the curdlan capsule is usually confined to cell aggregates or flocs. This observation leads to the hypothesis that abundant flocs ought to appear in curdlan producing cultures, but not in cultures incapable of curdlan production. Floc formation is assessed using a particle counter capable of detecting flocs as particles. (The particle counter detects flocs as particles using a cut-off of 10 and 40 µm, meaning that all flocs outside of this range, both larger and smaller, are excluded.) When grown in liquid minimal salts for 3 d on a rotary shaker, ATCC31749 contained a proportion of particles larger than 20 µm in diameter, while the *crdS* disruptant contained mostly particles with a diameter less than 20 µm (Fig. 2). At 6 days, the particles in the ATCC31749 culture are mostly larger than 20 µm in diameter. However, the particles from the *crdS* disruptant do not increase in diameter. This result supports the hypothesis that curdlan production is indeed associated with floc formation, and that the loss of curdlan production is associated with failure to form high numbers of flocs in liquid cultures.

### Floc formation allows continuous fermentation

It is likely that flocs form through the recruitment of capsulated cells, and through growth of new cells within a floc. Therefore, a versatile way of harvesting curdlan according to the invention would be to include differential sedimentation or filtration of the fermentation broth in such a way that the cells producing curdlan are separated from cells that have not yet begun curdlan production, which are free floating single bacteria. These can therefore continue to grow, and produce curdlan while the continuous removal of flocced bacteria will allow for longer fermentation times with fewer interruptions for harvesting curdlan.

### 96-well plate assay reveals onset of curdlan production.

A tight correlation *crdASC* gene expression and curdlan production would provide support for the idea that curdlan production is controlled by Agrobacterium at the level of *crdASC* gene expression. On the other hand, a poor correlation would indicate that curdlan production was controlled by additional unknown factors. A previous study using transcription analysis found a 100-fold upregulation of the *crdASC* genes upon the transition to nitrogen starvation, which lends weight to a tight correlation. To recapture this result, as well as to have a more sensitive, rapid and cheaper technique for the detection of both gene expression and curdlan production, a microplate assay capable of high throughput was developed. Previously, detection of curdlan production had been performed qualitatively via growth on agar containing aniline blue (where curdlan producing colonies turn intense blue while non-producing colonies remain white) or quantitatively following the extraction of curdlan via alkaline solution, neutralization, washing, drying and weighing. Growth on standard aniline blue agar plates is impractical for obtaining quantitative data, while the extraction method is relatively laborious and impractical for analyzing a high number of samples. For developing the microplate assay, advantage is taken of the knowledge that curdlan accumulation in bacterial cultures is easily detectable using the non-toxic (1→3)-β-glucan-specific dye aniline blue. For the setup, 100 µL of 1% agar containing aniline blue and nutrients are placed in the wells of a 96-well microplate. The rational for using agar (instead of liquid broth only) in the 96-well plate is to minimize the volume of liquid cultures. This reduces the problem of measuring growth in liquid cultures with strong floc formation and the associated variation between measurements. Once the agar is set, the bacteria are transferred to the agar, and the plate is covered with a transparent plastic lid to prevent drying of the agar and incubated in a Tecan reader at 30°C up to 72 hours, with automatic measurements at 3-hour intervals. The Tecan reader measures optical density, indicating bacterial growth, and fluorescence, indicating curdlan production.

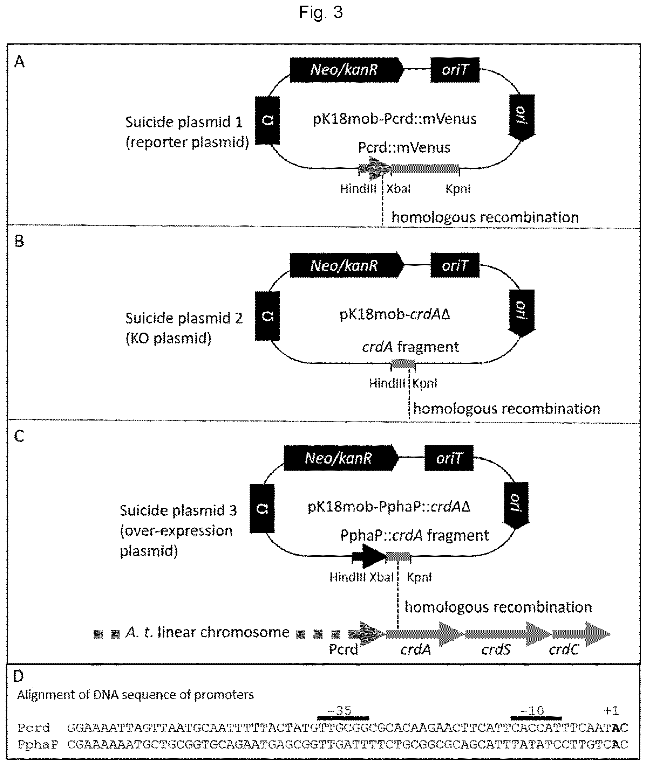

For the 96-well plate assay, reporter strains are prepared (using, for example, C58 and ATCC31749) in which the gene for mVenus is fused (translational fusion) to *crdA* (see Fig. 3 for details of the genetic construct). Use of these strains makes it possible for detecting correlation between the onset of *crdASC* gene expression (detected as mVenus fluorescence) and the onset of curdlan production (as detected via aniline blue/curdlan fluorescence).

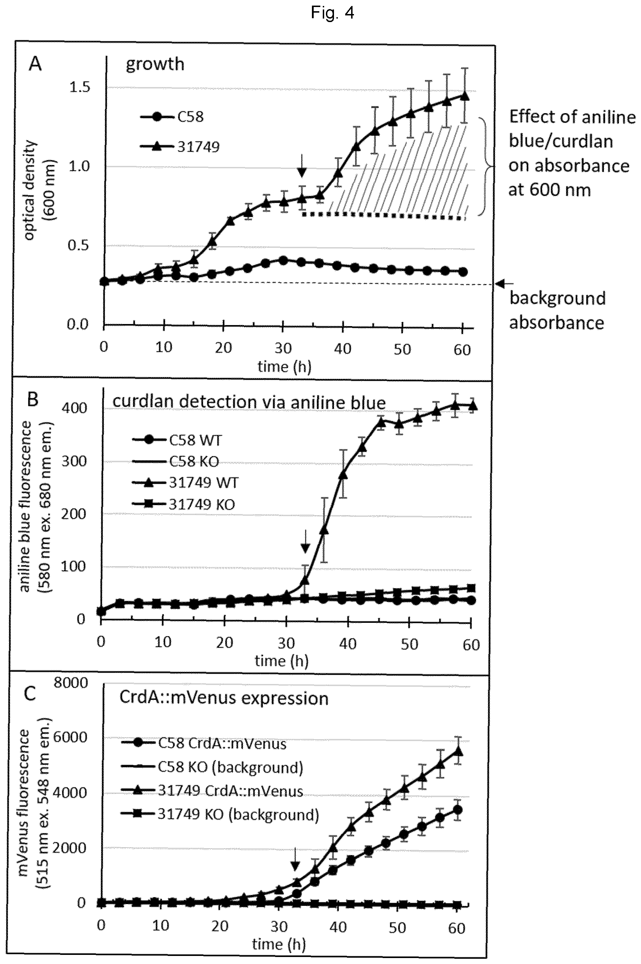

Absorbance at 600 nm (providing an indication of cell density, Fig. 4A) is strongly impacted by curdlan accumulation in strain ATCC31749 at around 30 hours, since binding between aniline blue and curdlan results in an intense blue colour that also absorbs light at 600 nm (Fig. 4A). The large error bars are likely because of the irregular colony surface that accompanies curdlan production. Growth of C58 was weaker than that of ATCC31749.

Curdlan binds to aniline blue, and the complex emits red fluorescence at 548 nm when excited by light at 515 nm. Fluorescence from the aniline blue-curdlan complex is apparent in cultures of ATCC31749 after 30 hours, but not in cultures of C58 (Fig. 4B). Interestingly, fluorescence from mVenus (Fig. 4C) is apparent in cultures of both ATCC31749 and C58 around 30 hours, although a weaker basal level of mVenus fluorescence is apparent slightly earlier, at 24 hrs, for ATCC31749.

It is known that the unmodified *Agrobacterium tumefaciens* strain C58 does not produce curdlan in the lab. Strain ATCC31749 is known to produce curdlan after about 72 hours under standard fermentation conditions. The data in Fig. 4 shows that curdlan production by ATCC31749 commences in the microplate assay at about 30 hours, but is completely lacking in C58. Interestingly, the expression of the *crdASC* genes is somewhat similar between the strains, in both cases also commencing at around 30 hours, albeit the expression in strain C58 is about 2-fold lower than in ATCC31749. Thus, the onset of *crdASC* gene expression correlates relatively well with the onset of curdlan accumulation in ATCC31749, but not in C58. Altogether, the newly developed assay appeared capable of sensitive and reliable detection of curdlan accumulation and *crdASC* gene expression at a high throughput level.

**The *Rhodobacter sphaeroides* promoter PphaP is much stronger than Pcrd in Agrobacterium.** Since curdlan production correlates with *crdASC* gene expression (at least in strain ACCT31749), one possibility for enhancing curdlan production is the enhanced expression of the *crdASC* genes. For this goal, the replacement of the native *crdASC* promoter (Pcrd) with a constitutively active promoter is sufficient, in the way as is revealed herein. Surprisingly, there is a candidate promoter discovered, as is revealed herein, during investigations on transcription regulation in the Alphaproteobacteria. This candidate is PphaP from *Rhodobacter sphaeroides,* which is very active in *Agrobacterium tumefaciens* C58, but could not be expected to be as active in other species. To compare the activity of PphaP with that of the native promoter of the *crdASC* genes, Pcrd, in Agrobacterium, the reporter gene for mVenus was fused to each of the promoters on the low copy plasmid pPHU231 (see section detailed embodiments below for details). The Agrobacterium strains C58 and 31749 carrying the KO plasmid are exemplarily used, since this avoids curdlan production and the resulting floc formation, which tends to interfere with fluorescence measurements and increase standard deviation. Furthermore, a variety of media is used. PY medium contains peptone and yeast extract (a rich source of nitrogen), while the MOPS medium contains a defined, growth limiting level of nitrogen. Apple extract (10%) - for example - represents a cheap and locally available source of nutrients in Germany. For experimental details see section detailed embodiments below. In particular, the waste associated with apple storage, transport and pulp from juicing might be used for curdlan production, for example in Germany. It is shown herein exemplarily that apple extract, added to agar at 10%, provided a high yield of curdlan. Thus, apples, resp. residues from processing apples within food industry, are an example of a cheap source of carbon for curdlan production. Combined with the new strains exemplarily developed in this study, revealed herein, that are not sensitive to nitrogen levels, new fermentation processes based on a variety of cheap plant biomass sources will dramatically decrease the cost of curdlan production, extending the use of curdlan in industry.

Other agricultural waste materials may be used as well without leaving the scope of the invention, for example sugar cane, orange peels, cassava starch, wheat bran, prairie cordgrass, and grass lawn cuttings. The bacterium in this invention, *Agrobacterium,* is known for its endophytic lifestyle, i.e., as a harmless inhabitant of a very broad range of plants, from grasses to trees. Some species of Agrobacterium can also cause crown gall on a very broad range of plants around the globe. The ability of these bacteria to colonize a broad range of plants suggests that this bacterium could convert carbon from a broad range of agricultural waste flows into curdlan. The invention here applies to agricultural waste from around the world, e.g., waste forms of vegetables and fruits from varied climates. This includes tomatoes, apples, grapes, strawberries, watermelons, tomatoes, oranges, mangoes, bananas, apples, pineapples and dates. Other carbon sources are also possible, for example ulvan, the predominant polysaccharide found in the common ocean algae Ulva. Ulvan consists of glucuronic acid, iduronic acid, xylose and rhamnose. These are released upon saccharification of ulvan, and can be directly used as fermentation sources for curdlan production. Another source of ulvan is the unwanted algal blooms that are increasingly prevalent in fresh and salt waters.

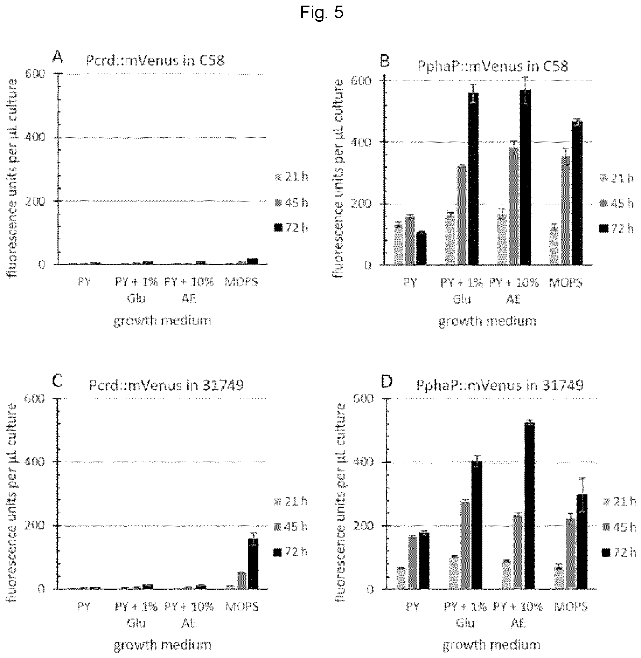

In C58, the activity of Pcrd is low during growth in PY (Fig. 5A) and also in MOPS medium. In contrast, the PphaP promoter activity is much stronger in all media (Fig. 5B). A similar pattern is observed with the Pcrd and PphaP promoters in 31749 (Fig. 5C & 5D). The Pcrd promoter is virtually inactive in PY medium, while in the MOPS medium, the Pcrd promoter is not active at 21 hours, but does show some activity at the 45 and 72-hour time points (Fig. 5C). This fits well with the data in Figure 4C (the main difference being that the crdA::mVenus fusion is carried on the chromosome in Fig. 4C, and on a low copy plasmid in Fig. 5). This also fits with the knowledge that the expression of the *crdASC* genes is strongly activated upon N depletion. In summary, these data demonstrate that PphaP surprisingly is far more active than Pcrd in all the growth media exemplarily tested, especially in both N-rich and N-depleted media.

**PphaP activates curdlan production.** The following experiments show how the PphaP promoter affects curdlan accumulation. Specifically, the use of PphaP drives the expression of *crdASC* leading to a higher production of curdlan. For testing this, the developed 96-well plate assay is used.

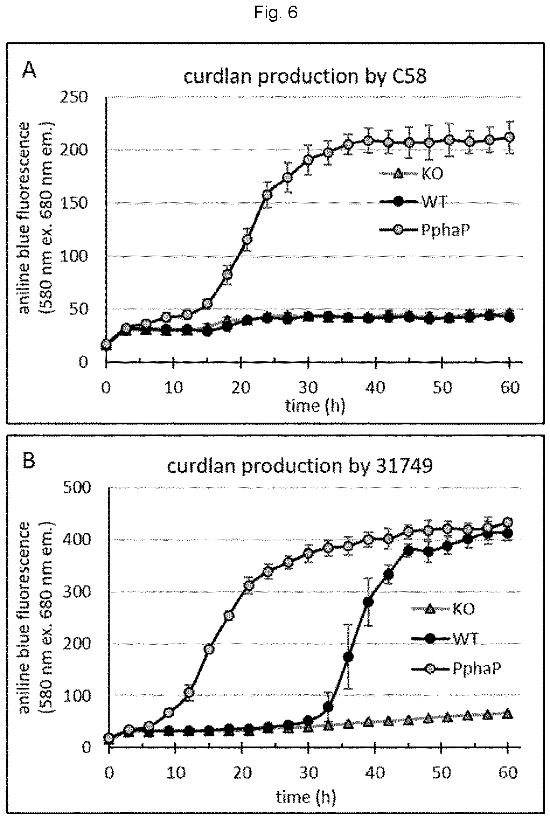

The data in Fig. 6 shows that the C58 WT (Fig. 6A) does not produce curdlan (as is also shown in Fig. 4B), while the insertion of PphaP into the C58 chromosome results in curdlan production. Also, in 31749 the presence of PphaP clearly enhances curdlan production so that curdlan accumulation is apparent much earlier, at around 10 hours of incubation, while in the 31749 WT, the onset of curdlan accumulation is apparent after 30 hours of incubation (Fig. 6B). Fluorescence reaches maximum levels at around 400 fluorescence units in 31749. Higher level were not observed, possibly because of exhaustion of the carbon source (mannitol).

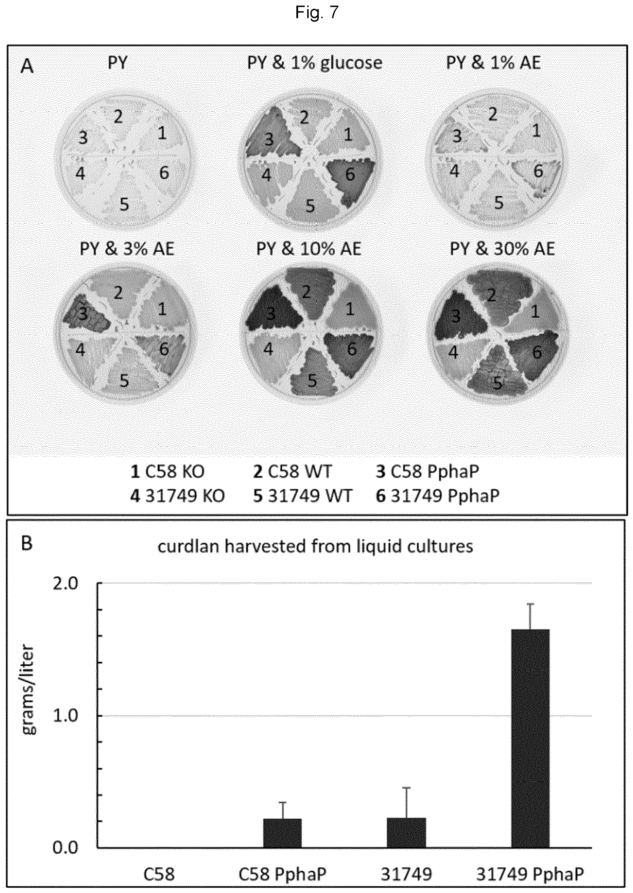

**Curdlan production requires both the expression of the *crdASC* genes and a suitable carbon source.** When grown on PY agar with aniline blue but without any additional carbon source, the colonies do not stain blue, indicating the absence of curdlan production (Fig. 7A). However, when glucose is added (e. g. 1% final concentration), both the strains carrying PphaP, but not the WT strains, stained blue during growth on agar, indicating curdlan production (Fig. 7A). This result shows that curdlan production requires at least two criteria: high expression of the *crdASC* genes as well as the presence of a suitable carbon source. Only one of these two conditions does not lead to curdlan production. Importantly, and unexpected from previous state of the art, when Pcrd is replaced with PphaP, the bacteria are capable of curdlan production regardless of whether nitrogen is limiting, provided that a suitable source of carbon is available.

**Apple extract is a suitable carbon source for curdlan production.** Given that one of the major costs associated with curdlan production is the carbon source, examples of realistic alternatives to the typical carbon sources were sought. Apple juice (100%) was obtained as one model example for manufacturing residues of juice-production from a local apple juice factory, sterilized via autoclaving and added to PY agar in increasing concentrations. At 1% (final concentration), a low level of curdlan production is apparent judging by the aniline blue stain (Fig. 7A). Production is improved with 3%, 10% and 30% apple extract, with 10% supporting the most intense blue colour formation.

To quantify curdlan, the bacteria are grown in liquid cultures for 3 days with ADB using glucose as a carbon source. The results (Fig. 7B) show that curdlan production by C58 is not detected after growth in ADB, while C58 PphaP produces about 0.1-0.2 g/L curdlan. Curdlan production by 31749 in ADB is also about 0.2 g/L, while production by 31749 PphaP in ADB reaches 1.6 g/L. Thus, under these exemplary conditions, PphaP improves curdlan production about 8-fold in strain 31749 and forced C58 to produce about 0.2 g/L curdlan under conditions where it would normally not produce any (Fig. 7B).

Curdlan-producing Agrobacterium colonies grown on agar medium with a sufficient carbon source and aniline blue produce curdlan as a stable, intensely blue-staining layer which may be stripped, along with the cells, from the colony surface. This suggests - without being bound to a certain theory - close proximity between cells and curdlan, and that curdlan is somehow adhesive and involved in attachment between cells. However, the relationship between curdlan and the bacterial cell surface had not been clearly defined. Scanning electron microscopy of the Gram positive curdlan producing bacterium *Cellulomonas* indicated that curdlan may be deposited as a capsule. However, staining with aniline blue could not support a capsular formation by *Cellulomonas* since the native form of curdlan produced by this bacterium does not stain with aniline blue. It must first undergo alkaline extraction before staining, indicating a fundamentally different native form to curdlan produced by Agrobacterium. Herein it is shown that Agrobacterium deposits curdlan as a capsule, using the (1→3)-β-glucan-specific aniline blue fluorochrome (ABF). Additionally, curdlan is negatively-stained by uranyl acetate and thereby visible using TEM. Both ABF-induced fluorescence images and TEM show that the curdlan capsule is tightly associated with the cell surface. Neither of these features was observed with a curdlan deficient mutant (*crdS*-) or in cultures of strains grown in medium that does not elicit curdlan production, i.e., Luria broth.

Without being bound to a certain theory, it is considered that curdlan-production has the following effect: Cell aggregates depend upon curdlan production, and these aggregates appear to be via interactions between curdlan-encapsulated cells. The data is consistent with a scenario where cells produce a capsule, whereupon these encapsulated cells are recruited to the aggregate on the basis of curdlan-curdlan interactions. In other words, after producing curdlan, the cell randomly encounters a neighbouring cell that has also produced curdlan. The two become attached and then encounter additional cells that have produced curdlan, and in this way the aggregates grow larger through cell recruitment over time (Fig. 2). Studies on the soil-dwelling Gram-positive *C*. *flavigena* also found a correlation between aggregation and curdlan production. Furthermore, on subculturing *C. flavigena* on a nitrogen-rich medium lacking a carbohydrate source, disaggregation occurs and the curdlan disappears, suggesting that the curdlan is mobilized as a carbohydrate source. Consumption of curdlan by Agrobacterium has not been observed.

*Agrobacterium tumefaciens* is considered one of the more important, well-studied plant pathogens, being a tumorigenic pathogen responsible for crown gall disease with a wide host range. So far, there is no indication that curdlan is necessary for pathogenesis. Agrobacteria strains are endemic in soils. Many are not necessarily pathogenic, and may exhibit long term survival even in the absence of host colonization. Probably the ability to produce curdlan is related to long term survival, although this has never been demonstrated. Indeed, the *Agrobacterium tumefaciens* C58 strain used in this study did not produce curdlan under the conditions tested, with the only exceptions being when C58 was grown on apple extract, carried PphaP, or both (Fig. 7A). It is likely that curdlan production is more highly regulated in C58 than in 31749 and that, in addition to nitrogen limitation, other environmental conditions (such as desiccation, high oxygen levels or plant-derived molecules) are needed for the activation of curdlan production in C58. Thus far, the conditions that initiate curdlan production in strain C58 are unknown, and more research is needed to understand the role of curdlan in the bacterial lifestyle.

The curdlan dependent aggregation phenomenon described herein appear to enable the formation of microcolonies or flocs and biofilms in these nutritionally stressful situations. The curdlan capsule formed under these conditions may play a role in protection from desiccation by virtue of the impressive water-holding capacity of curdlan capsule, i.e., the encapsulated cell may act as a type of spore, as already suggested within state of the art in the case of cellulose-producing bacteria. Moreover, curdlan might protect not only the producing cells, but also the entire community of cells from desiccation as well as soil predators such as phagocytic protozoans and parasitic Bdellovibrio species.

### PphaP is a suitably strong promoter but not alone sufficient for enhancing curdlan production

The *phaP* promoter (PphaP) from *Rhodobactersphaeroides* controls the expression of the phasin gene *phaP.* Previous work on this gene found that it was strongly active in the exponential growth phase of *Rhodobacter sphaeroides.* Interestingly, upon heat shock, transcription of the *phaP* gene increased by over 20-fold, making the promoter of *phaP* one of the most active promoters that have ever been observed. Indeed, PphaP surprisingly shows very high activity in Agrobacterium strains (Fig. 5). In comparison, the native *crd* promoter (Pcrd) is not only much weaker than PphaP, it is also highly dependent upon the growth medium (e.g., nitrogen content). For example, PY medium (containing peptone and yeast extract) does not support Pcrd activity, while PphaP is strongly active. This makes PphaP the ideal candidate for strong, unregulated expression of the *crdASC* genes. The outcome of this approach is a clear improvement in curdlan production (Fig. 6 & 7). However, curdlan production by strains carrying PphaP remains dependent upon the presence of a suitable carbon source (Fig. 7A). This outcome shows that curdlan production is not only dependent upon expression of the *crdASC* genes, but also upon the presence of a suitable carbon source, which determines the metabolic status of the cell.

It is important that the KO strains do not produce curdlan (Fig. 6 & 7A). The significance here is that the suicide plasmid 2 (see Fig. 3B) does not disrupt the *crdASC* genes upon insertion into the Agrobacterium chromosome. Rather, the insertion causes transcription from Pcrd to be terminated by a transcription terminator, and thus the *crdASC* genes in the KO strains are not disrupted but rather isolated from Pcrd via the terminator. The outcome is that the KO strains do not produce curdlan, even in the presence of a suitable carbon source (Fig. 7A). This fits with the explanation that the *crdASC* gene cluster is indeed an operon with Pcrd as the promoter of the operon. Moreover, curdlan production requires both criteria: *crdASC* expression and a suitable carbon source. Nitrogen limitation is not necessary, so long as these two requirements are satisfied. This technical effect is therefore ideal for providing the subject matter of the invention, being disclosed herein. Despite genetically fixing the high *crdASC* gene expression, curdlan production will only follow once the bacterium encounters an adequate carbon source. This is useful for preparing starter cultures that are not encumbered with excessive curdlan production and cell aggregation.

Figure 7A provides exemplary qualitative data about stability of strains being modified with a suicide vector. Herein, the presence of pK18mob2 in the chromosome is useful for providing a kanamycin selection marker as well as a means for checking the kanamycin resistance status of any spontaneous reverting mutants (i.e., those mutants who spontaneously lose the suicide plasmid through homologous recombination, essentially the same process as plasmid integration but in reverse). The appearance and persistence of such reverting mutants would provide an indication of any selection advantage associated with the loss of the suicide plasmid. In this experiment, the KO strains (white colonies) were grown on agar lacking the antibiotic kanamycin. A spontaneous loss of this suicide plasmid would result in the appearance of blue colonies as cells recovered the ability to produce curdlan when grown on agar containing a suitable carbon source. Thus, the presence of blue colonies mixed with white colonies would provide a qualitative measurement of the stability of the suicide vectors. That no blue colonies appeared among the KO strains is an indication that the suicide plasmid is highly stable. It is also worth noting that if, for some reason, the presence of the suicide vector in the chromosome of the curdlan producing strain is undesirable, its removal is possible (while maintaining the presence of the strong constitutive promoter, e.g. PphaP) through a well-established method that uses, for example, the sacB gene. Thus, the presence of a suicide vector is no essential part of the invention - it may be part of the invention or it may not be part of the invention. The suicide vector, resp. suicide plasmid is exemplarily used as carrier for the insertion of the strong constitutive promoter; other means of inserting the promoter-sequence can be used also (e.g. CRISPR/Cas9) without leaving the scope of the invention.

### Critical parameters for obtaining maximal curdlan production

A large body of literature covers the parameters for obtaining maximal curdlan production. This includes specifications of temperature, pH, oxygen levels, levels of various other macronutrients (e.g., carbon, nitrogen, phosphorous), as well as fermentation times. These parameters may need to be established for the bacterial strains that have been genetically modified using this invention.

The following DNA-sequences are examples of sequences being comprised by the scope of the invention.

### DNA sequences relevant for suicide and reporter plasmids

(Upper Case: General sequences, Lower Case: translation starts (atg), restriction digest sites, e.g., tctaga for Xbal)

### List of components of plasmids:

- pK18mob2 (incomplete sequence, full sequence available at NCBI)
- *crdA* promoter region sequence (also referred to as Pcrd)
- *crdA* coding region, partial (not complete)
- mVenus coding region
- *phaP* promoter region (also referred to as PphaP)

SEQ ID 1:
   pK18PcrdA-mV, Suicide plasmid 1a (reporter plasmid for 31749), origin: compiled from various organisms, PcrdA (Agrobacterium sp. 31749), mVenus (Aequorea victoria), plasmid pK18mob2, synthetic. 
ACGACGGCCAGTGCCaagcttAGTTTTGGGAAAAGCAACGGCACTTCCATACC
GACTTGAGGCGCTAACGCGCTTGCGGTCTGGAACTTGACCTACCCGGCGGT
 
AGGTCTTTGCGACGGCATGCCGTCGCCCCCATAAGTTGCATATGCGAAATGG
CGAAGCCAGGGTGTGTTTTCCAAGCACGCCATTTAAAAGGCAATTCTAATATG
AAACGATTGAAATTTCGTCTTTCAAATTCTCTTCGATGAATTTTCGCGTCCCCA
AAATCGTCAGATGCCTATTTGTTGGTCAAAATTGGAAAATTAGTTAATGCAATT
TTTACTATGTTGCGGCGCACAAGAACTTCATTCACCATTTCAATACTGCGGGA
GGAACGGTTTCTCAACAACAGAGGAGTTGATTTCatgCTGTTCCGCAATAAGC
CTGACGCAAAATATAAAGGCGCATCCGTAAGAtctagaatgGTGAGCAAGGGCGA
GGAGCTGTTCACCGGGGTGGTGCCCATCCTGGTCGAGCTGGACGGCGACGT
AAACGGCCACAAGTTCAGCGTGTCCGGCGAGGGCGAGGGCGATGCCACCTA
CGGCAAGCTGACCCTGAAGCTGATCTGCACCACCGGCAAGCTGCCCGTGCC
CTGGCCCACCCTCGTGACCACCCTGGGCTACGGCCTGCAGTGCTTCGCCCG
CTACCCCGACCACATGAAGCAGCACGACTTCTTCAAGTCCGCCATGCCCGAA
GGCTACGTCCAGGAGCGCACCATCTTCTTCAAGGACGACGGCAACTACAAGA
CCCGCGCCGAGGTGAAGTTCGAGGGCGACACCCTGGTGAACCGCATCGAGC
TGAAGGGCATCGACTTCAAGGAGGACGGCAACATCCTGGGGCACAAGCTGG
AGTACAACTACAACAGCCACAACGTCTATATCACCGCCGACAAGCAGAAGAA
CGGCATCAAGGCCAACTTCAAGATCCGCCACAACATCGAGGACGGCGGCGT
GCAGCTCGCCGACCACTACCAGCAGAACACCCCCATCGGCGACGGCCCCGT
GCTGCTGCCCGACAACCACTACCTGAGCTACCAGTCCAAACTGAGCAAAGAC
CCCAACGAGAAGCGCGATCACATGGTCCTGCTGGAGTTCGTGACCGCCGCC
GGGATCACTCTCGGCATGGACGAGCTGTACAAGTAAgaattcGTA
SEQ ID 2:
   pK18PcrdA-mV, Suicide plasmid 1b (reporter plasmid for C58), origin: compiled from various organisms, PcrdA (*Agrobacterium tumefaciens* strain C58), mVenus (Aequorea victoria), plasmid pK18mob2, synthetic. 
ACGACGGCCAGTGCCaagcttAGTTTTGGGAAAAGCAACGGCACTTCCATACC
GACTTGAGGCGCTAACGCGCTTGCAGTCTGGAAATTGACCTACCCGGCGGC
AGGCCTTTGCGACGGCATGCCGTCGCCCCCATAAGTTGCATATGCGAAATGG
CGAAGCCAGGGTGTGTTTTCCAAGCACGCCATTTAAAAGGCAATTCTAATATG
AAACGATTGAAATTTCGTCTTTCAAATTCTCTTCGATGAATTTTCGCGTCCCCA
AAATCGTCAGATGCCTATTTGTTGGTCAAAATTGGAAAATTAGTTAATGCAATT
TTTACTATGTTGCGGCGCACAAGAACTTCATTCACCATTTCAATaCTGCGGGA
GGAACGGTTTCTCAACAACAGAGGAGTTGATTTCatgCTGTTCCGCAATAAGC
 
CTGACGCAAAATATAAAGGCGCATCCGTAAGAtctagaatgGTGAGCAAGGGCGA
GGAGCTGTTCACCGGGGTGGTGCCCATCCTGGTCGAGCTGGACGGCGACGT
AAACGGCCACAAGTTCAGCGTGTCCGGCGAGGGCGAGGGCGATGCCACCTA
CGGCAAGCTGACCCTGAAGCTGATCTGCACCACCGGCAAGCTGCCCGTGCC
CTGGCCCACCCTCGTGACCACCCTGGGCTACGGCCTGCAGTGCTTCGCCCG
CTACCCCGACCACATGAAGCAGCACGACTTCTTCAAGTCCGCCATGCCCGAA
GGCTACGTCCAGGAGCGCACCATCTTCTTCAAGGACGACGGCAACTACAAGA
CCCGCGCCGAGGTGAAGTTCGAGGGCGACACCCTGGTGAACCGCATCGAGC
TGAAGGGCATCGACTTCAAGGAGGACGGCAACATCCTGGGGCACAAGCTGG
AGTACAACTACAACAGCCACAACGTCTATATCACCGCCGACAAGCAGAAGAA
CGGCATCAAGGCCAACTTCAAGATCCGCCACAACATCGAGGACGGCGGCGT
GCAGCTCGCCGACCACTACCAGCAGAACACCCCCATCGGCGACGGCCCCGT
GCTGCTGCCCGACAACCACTACCTGAGCTACCAGTCCAAACTGAGCAAAGAC
CCCAACGAGAAGCGCGATCACATGGTCCTGCTGGAGTTCGTGACCGCCGCC
GGGATCACTCTCGGCATGGACGAGCTGTACAAGTAAgaattcGTA
SEQ ID 3:
   pK18-crdA KO, Suicide plasmid 2 (KO plasmid for C58), origin: *Agrobacterium tumefaciens* C58, plasmid pK18mob2, synthetic. 
ACGACGGCCAGTGCCAAGCTTGCATGCCTGCAGgtcgacAACAGAGGAGTTGA
TTTCatgCTGTTCCGCAATAAGCCTGACGCAAAATATAAAGGCGCATCCGTAAG
ATTAGCCTCCTATACTGTTCTCGGTGTGGTGGCGACGGTGTTTTCTTCTTCCG
TGTTTCCGCCTGTATTCACCTCTCAAACGAGCCGTGCTCTGATCAATGCCCCC
ATTATTCCCTTGACGACGCCGATCAGCGGCGTTGTGAAAACATCGAACGACA
GGGGCGGTGCCACCATCGAGAACGACAAGGTGGACAATACAACGCTGATCG
GCCTGAAGGTTCAACTGGCGGCGCTTGACAATGAGATGCGCCAGAAGAACT
CGATCGTTTCGGACTATGCCTCGCGGATCGACGACCTGAACAGGGATCTTTC
CAGCCAGCAGGCGGCTTTGCTGCTGCGGACAGAGTCCGATCTCGAGGCTGC
AGAGGCGGCCTTGCAGATGGTTACCTATTCCACGCGGATCGCAAAAGCCGAA
GCGGCTCGCAAACAAAAACTCGTCtctagaGGATCCCCGGGTACCGAGCTCGA
ATTCGTA
SEQ ID 4:
   pK18-PphaP-crdA, Suicide plasmid 3 (over-expression plasmid for 31749), origin: Agrobacterium sp. 31749, *Rhodobacter sphaeroides,* plasmid pK18mob2, synthetic.

aagcttGCAACGCAAGGCGTTTCGGCCAGGTGCGAAAAATGCTGCGGTGCA
GAATGAGCGGTTGATTTCTGCGGCGCAGCATTTATATCCTTGTCACAGGATA
ACCGGGCATGAGCCCGCTCGCAGGAGAgtcgacAACAGAGGAGTTGATTTCatg
CTGTTCCGCAATAAGCCTGACGCAAAATATAAAGGCGCATCCGTAAGATTAG
CCTCCTATACTGTTCTCGGTGTGGTGGCGACGGTGTTTCTTCTTCCGTGTTT
CCGCCTGTATTCACCTCTCAAACGAGCCGTGCTCTGATCAATGCCCCCATTAT
TCCCTTGACGACGCCGATCAGCGGCGTTGTGAAAACATCGAACGACAGGGG
CGGTGCCACCATCGAGAACGACAAGGTGGACAATACAACGCTGATCGGCCT
GAAGGTTCAACTGGCGGCGCTTGACAATGAGATGCGCCAGAAGAACTCGATC
GTTTCGGACTATGCCTCGCGGATCGACGACCTGAACAGGGATCTTTCCAGCC
AGCAGGCGGCTTTGCTGCTGCGGACAGAGTCCGATCTCGAGGCTGCAGAGG
CGGCCTTGCAGATGGTTACCTATTCCACGCGGATCGCAAAAGCCGAAGCGG
CTCGCAAACAAAAACTCGTCtctagaGGATCCCCGGGTACCGAGCTCGAATTCG
TA

Thus, the invention can be summarized as follows:
The invention comprises a transcription overriding cassette (TORC) which is constructed for obtaining maximal expression of the at least one gene of interest, namely the cluster of genes of interest comprising: *crdA, crdS* and *crdD.* The expression *overriding* herein means that the insertion of promoter PphaP into *Agrobacterium* surprisingly has the following effects: PphaP is (within *Agrobacterium*) firstly, constantly active (i. e. there is no existing regulation or control over its state of activity when in *Agrobacterium* ), secondly, the constant activity is at a much higher level than that of the native promoter of the *crdASC* gene cluster, and, thirdly, its activity is unexpectedly much higher in *Agrobacterium* than in *Rhodobacter sphaeroides,* its natural host-bacteria. This effect (that the promoter of a certain bacterial species is becoming constantly much more active when transferred into another bacterial species) is an unexpected one, not yet known within state of the art heretofore. The TORC is characterised in that it is comprised of two parts, a first DNA sequence comprising the phaP promoter DNA sequence and a second DNA sequence comprising at least one fragment from the at least one gene of interest, in this case specified as *crdA.* The fragment from the at least one gene of interest is comprising at most the first 1500 nucleotides of the at least one gene of interest, preferredly the first 1000 nucleotides of the at least one gene of interest, more preferredly the first 500 nucleotides of the at least one gene of interest and may even be reduced to only the first 300 nucleotides of the at least one gene of interest. The first DNA sequence (strong constitutive promoter) is located directly before the second DNA sequence (first part of the at least one gene of interest, as described just above) within the TORC and the first and second DNA sequences are connected directly to each other within the complete sequence of the TORC or are separated from each other within the complete sequence of the TORC by DNA sequences of DNA restriction cleavage sites. The size of the fragment of the at least one gene of interest has influence on the stability of the genetic modification introduced into the bacterial cell: With decreasing size the stability of the genetic modification increases. While the part of the at least one gene of interest is absolutely necessary for the invention, the restriction sites are only useful for the construction of the TORC. The inclusion of the TORC in a suicide plasmid is useful for introducing the genetic modification into the host cell chromosome. However, following the introduction, the suicide plasmid is not necessary for the functioning of the TORC (if the method via suicide plasmid is applied), and can be removed after having inserted the strong constitutive promoter and the first at least 300 nucleotides of the at least one gene of interest. A special difficulty with the part of the at least one gene of interest is that the smaller the sequence becomes, the more difficult it is to be introduced into the host chromosome via homologous recombination.

The invention further comprises a transcription overriding cassette (TORC) as described above, wherein the first DNA sequence comprises a promoter DNA sequence which has at least 50% nucleotide identity with the promoter DNA sequence according to SEQ ID 6 and the second DNA sequence comprises at least one fragment from the at least one gene of interest which is a DNA sequence having at least 50% nucleotide identity with SEQ ID 23, SEQ ID 23 being *crdA* gene partial fragment (507 bp) from *Agrobacterium tumefaciens* C58 (natural sequence, 18 bp upstream of start codon plus the first 489 bp of the coding region of *crdA*):

AACAGAGGAGTTGATTTCatgCTGTTCCGCAATAAGCCTGACGCAAAATATAAA
GGCGCATCCGTAAGATTAGCCTCCTATACTGTTCTCGGTGTGGTGGCGACGG
TGTTTTCTTCTTCCGTGTTTCCGCCTGTATTCACCTCTCAAACGAGCCGTGCT
CTGATCAATGCCCCCATTATTCCCTTGACGACGCCGATCAGCGGCGTTGTGA
AAACATCGAACGACAGGGGCGGTGCCACCATCGAGAACGACAAGGTGGACA
ATACAACGCTGATCGGCCTGAAGGTTCAACTGGCGGCGCTTGACAATGAGAT
GCGCCAGAAGAACTCGATCGTTTCGGACTATGCCTCGCGGATCGACGACCT
GAACAGGGATCTTTCCAGCCAGCAGGCGGCTTTGCTGCTGCGGACAGAGTC
CGATCTCGAGGCTGCAGAGGCGGCCTTGCAGATGGTTACCTATTCCACGCG
GATCGCAAAAGCCGAAGCGGCTCGCAAACAAAAACTCGTC

The invention further comprises a transcription overriding cassette (TORC) having a DNA sequence of the TORC which has at least 50% nucleotide identity with SEQ ID 4.

The invention further comprises a vector comprising at least one variant of the TORC as is described above.

The invention further comprises the usage of a variant of the TORC as described above for the homologous or heterologous expression of at least one gene of interest in cells by transforming the TORC into these cells and cultivating them, whereat the promoter of the TORC is replacing the natural promoter of the at least one gene of interest.

The invention further comprises the usage as described above, wherein the at least one gene of interest is encoding at least one enzyme for the production of curdlan.

The invention further comprises a recombinant bacterial cell of an Agrobacterium, comprising a variant of the TORC as described above and the corresponding at least one gene of interest, whereat the at least one gene of interest is operatively linked, i. e. covalently bound, to the TORC and both, the TORC and the at least one gene of interest, are located within the bacterial chromosome.

The invention further comprises the bacterial cell as described in the previous paragraph, characterised in that it is a cell of *Agrobacterium sp.* strain ATCC 31749 or *Agrobacterium tumefaciens.* An alternative name for *Agrobacterium tumefaciens* found in the literature is *Rhizobium radiobacter.*

The invention further comprises a method for transformation of a cell of an Agrobacterium with a variant of the TORC as described above by
a) providing a variant of the TORC as described above;
b) transformation of an Agrobacterium with the TORC;
d) selection of the host cells that have been stably transformed with the TORC by using the respective selection agents;
e) isolating the transformed host cells.

The invention further comprises a method of producing curdlan, characterized in that it comprises the following steps:
a) providing cells of an Agrobacterium as identified above and a fermentation broth suitable for growing cells of the Agrobacterium as identified above;
b) mixing the Agrobacteria and the fermentation broth according to step a) and performing a fermentation;
c) gathering the curdlan having been produced during step b) at least partially.

The method of producing curdlan, revealed in the preceding paragraph may further comprise as additional step d) purifying the curdlan gathered in step c). This step d) may not be necessary in case that the curdlan, having been gathered via step c), is already pure enough for the intended application, e. g. for usage as supplement in super-processable concrete.

The invention further comprises the method as described above, wherein step c) comprises filtering the fermentation broth through a filter having a pore size which allows the separation of flocks of bacteria which have already produced curdlan from bacteria which have not yet produced curdlan, the pore size being within the range of 0.03 mm - 5.0 mm, more preferred within the range of 0.04 mm - 2.0 mm, most preferred within the range of 0.05 mm - 1.0 mm.

The invention further comprises the method as is described in the previous paragraph, characterized in that step c) comprises the performance of a sedimentation process upon the fermentation broth until the flocks of bacteria which have already produced curdlan are settled down and the bacteria which have not yet produced curdlan are still floating, the means for performing the sedimentation process being either centrifugation or subjecting the fermentation broth to natural gravity. In case of performing the separation process via centrifugation the centrifugation can be performed at broad ranges of acceleration force and time span (e. g. within 500 g to 10000 g and in between 1 s to 1 hour). If the separation process is performed via subjecting to natural gravity, this may be performed for a time span in between 5 minutes to 10 days.

### Detailed embodiments of the invention

### Materials and Methods:

**Bacterial strains and plasmids.** The strains exemplarily used herein are the curdlan producing Agrobacterium sp. strain ATCC 31749, whose genome has been sequenced and *Agrobacterium tumefaciens* C58. Mutants are exemplarily generated using a chromosomally inserted suicide plasmid (pK18mob2). For transferring plasmids into the Agrobacteria, exemplarily the *E*. *coli* strain S17-1 is used for conjugation. Plasmid construction is exemplarily performed using the restriction digest sites indicated in Fig. 3.

Upon homologous recombination with the chromosome, the suicide plasmid inserts an additional copy of the homologous fragment carried on the plasmid. For this approach, the different fragments from the *crdASC* gene cluster are used. Firstly, to learn about the expression of this cluster, a copy of the promoter region of *crdA* (upstream 300 bp) plus the first 51 bp of the coding region of *crdA* (351 bp total) is fused to the gene for the reporter protein mVenus. This translational fusion construct is inserted into the chromosome using the suicide vector pK18mob2 and homologous recombination (see Fig. 3A, suicide plasmid 1, reporter plasmid). Upon insertion, the CrdA::mVenus fusion provides a readout of *crdA* expression.

Another fragment of *crdA* is used in the second and third suicide variants (see Fig. 3B and 3C, suicide plasmids 2 and 3). For suicide plasmid 3, this *crdA* fragment (489 bp) is preceded by a fragment from the bacterium *Rhodobacter sphaeroides* encoding the *phaP* promoter (PphaP, 127 bp). Upon homologous recombination with the chromosome, this suicide plasmid variant inserts PphaP upstream of the *crdASC* gene cluster, meaning that expression of the cluster is now dependent upon PphaP.

For the measurement of the activity of Pcrd and PphaP using the reporter gene for mVenus, the low copy plasmid pPHU231 is exemplarily used in which either Pcrd or PphaP is fused to the gene for mVenus. In the case of Pcrd::mVenus, the fusion is a translational fusion, and is the identical fragment which is used for the suicide vector pK18mob-Pcrd::mVenus (see Fig. 3A). This fragment includes the ribosome-binding site of *crdA* plus the first 27 codons of *crdA* (including the translational start). In the case of PphaP::mVenus, the fusion included the native ribosome binding site of *phaP* plus the first 13 codons of *phaP* (including the translational start). A comparison of the two ribosome binding sites reveals some similarity:
CAGGAGACAAGGCAatg (SEQ ID 24) *for phaP* and
GAGGAGTTGATTTCatg (SEQ ID 25) for *crdA* (ribosome binding sites are written in bigger letters).

**Media and culture conditions.** Agrobacterium strains are - for example - routinely grown at 30°C for a minimum of 3 d on aniline blue agar (ABA). For liquid cultures (shaken at 130 rpm), strains are grown in Luria broth (LB, 10% Oxoid bacto-tryptone, 5% Oxoid yeast extract, 10% NaCl) and Agrobacterium defined broth (ADB) containing 20 mM KNOs, 2.8 mM K₂HPO₄, 12.8 mM KH₂PO₄, 11.5 mM Na₂SO₄, 1.25 mM MgCl₂, 0.1 CaCl₂, 0.09 mM FeCl₃, 0.05 mM MnCl₂, 1.0 mM citric acid and adjusted to pH 7.0 prior to autoclaving. Sterile D-glucose or D-mannitol (4% w/v final) is then added as a carbon source. The volume of the ADB cultures is 50 mL, grown on a rotary shaker at 350 rpm in 150 mL conical flasks plugged with cotton wool.

**Microscopy.** Fluorescence microscopy: An Olympus BX50 microscope with a xenon lamp and on a Zeiss Universal light microscope with ultraviolet light (365 nm from an OSRAM HBO-100 lamp) is used. Images are captured with an RT Monochrome SPOT camera (Diagnostic Instruments Inc.). Complexes of curdlan with the (1→3)-β-glucan specific, Aniline Blue Fluorochrome (ABF) (Biosupplies Australia Pty. Ltd.) are detected by mixing one drop of a 7 day culture from ADB with one drop of a 0.01% ABF solution on a glass slide, covering with a coverslip, which was then firmly pressed onto the slide. Fluorescence from the ABF-curdlan complex is observed with excitation at 330-385 nm and emission at 420-475 nm. For vital staining: 10 ml of a 4d-ADB culture is incubated with 5 mM 5-cyano-2,3-ditolyl tetrazolium chloride (CTC) for 1 h on a shaker (130 rpm) at room temperature. A drop of the culture is prepared as described above for fluorescence of ABF-curdlan complexes. Intracellular CTC fluorescence is observed with excitation at 530-550 nm and emission at 590-610 nm using the same optics as described above.

**Electron microscopy.** Cells from ADB cultures are harvested and washed in water by centrifugation at forces not exceeding 500 g. Samples are mounted on formvar-coated, 300-mesh copper grids, and negatively stained with 2.5% uranyl acetate for observations using a Philips 420 transmission electron microscope (TEM) at 100 kV. Images are captured with a GATAN camera, with a 20x magnification factor. For cell-sections, colonies are harvested from ABA and fixed in a solution of 5% glutaraldehyde and 4% para-aldehyde in 0.2 M calcium cacodylate, 0.1 M K₂HP0₃ (pH 7.0), for one day, washed in 0.1 M cacodylate, and post-fixed in 1% osmium tetroxide in 0.1 M cacodylate. The cells are dehydrated in a graded series of ethanol (50-100%) each for 15 min, followed by 100% acetone, infiltrated in a 25% solution of Spurr resin (Electron Microscopy Science) in acetone for 1 h, followed by 1 h incubations in 50% and 75% resin. Specimens are finally embedded in 100% resin overnight, and the resin polymerized at 60°C for 30 h. Specimen blocks are sectioned at 0.5 µm thickness, stained with lead citrate-uranyl acetate and viewed under a Philips 420 TEM at 100 kV.

**Particle counting.** ADB cultures grown for 2 to 7 d are diluted (1 in 10) with 50 mM Na₂HP0₄/NaH₂P0₄ (pH 7.0) before analysing with a particle counter/sizer (CDA-500, Model: F-520p, Sysmex) with a 100 gm aperture detector. Particles are measured at two ranges: 10-40 µm and 40-100 µm.

**96-well plate assay.** Sterile, transparent, flat-bottom 96-well microplates (Greiner Bio-One) are prepared by adding 100 µL of 1% agar (liquid, 60°C) containing aniline blue and nutrients from ADB or MOPS medium to each well. After the agar has cooled to room temperature, 20 µL of ADB or MOPS medium inoculated with Agrobacterium is pipetted onto the agar, and the plate is covered with a transparent plastic lid (with condensation rings, high profile, clear, sterile, Greiner Bio-One) and incubated in a Tecan reader (Infinite 200Pro (M Nano+)) at 30°C up to 72 hours. The Tecan reader is programmed to measure the cultures growing in the well automatically at 3 hour intervals.

**Curdlan extraction.** Curdlan is exemplarily harvested from liquid cultures of Agrobacterium as follows: The liquid culture (1 L) is subjected to centrifugation under various conditions, for example at 4000 x g, 10 min. The supernatant is discarded and the pellet is resuspended in a suitable amount of water, for example 1 L. NaOH or a similar base (for example KOH, ammonia, calcium hydroxide or an organic base) is added to the cell suspension until a final concentration of - for example - 0.5 M is reached, and stirred for a suitable amount of time, for example 5 minutes. The cells are removed by centrifugation (for example at 10000 x g for 10 minutes). The supernatant is removed to a clean container carefully without disturbing the cell pellet. An acid, for example HCl (32%) is added slowly to the supernatant, for example in a step-wise fashion, with good mixing, until about pH 7. Any other suitable acid can also be used, for example acetic acid, nitric acid, sulfuric acid etc. Then the neutralized suspension is centrifuged, for example at 10000 x g for 10 min. The supernatant is carefully removed and discarded. The almost-transparent curdlan pellet is washed by resuspending in water and centrifugation, for example at 10000 x g. This step may be repeated several times, for example 3 times, before the curdlan is placed in a suitable container, for example a Petri-dish and incubated at 40-50°C for 2-3 days (or any other temperature and time between -20 °C and 100 °C and 1 hour and 10 days). The dried curdlan is then weighed. It is well known to any person, skilled in the art, that the parameters described herein are just examples for growing the bacteria and extracting the curdlan, and can be varied within a broad range without leaving the scope of the invention.

**Media for curdlan production.** ADB (Agrobacterium defined broth) is medium that is weakly buffered with a phosphate solution, so that initial growth of Agrobacterium is at around pH 7, which then subsequently drops to around pH 5.5 at the onset of curdlan production. ADB consists of - for example -4% (w/v) glucose, 20 mM KNOs, 2.8 mM K₂HPO₄, 12.8 mM KH₂SO₄, 11.5 mM Na₂SO₄, 1.25 mM MgCl₂, 0.09 mM FeCl₃, 0.05 mM MnCl₂, 1.0 mM citric acid, adjusted to pH 7.0 with a base (e.g. NaOH) before autoclaving. Sterile glucose may be added to the salts solution after autoclaving.

MOPS-buffered medium is previously used for growth of *Sinorhizobium meliloti* in a defined medium and also supports curdlan production by Agrobacterium. The MOPS medium consists - for example - of 50 mM MOPS (adjusted to pH 7 with a base, e.g. KOH), 55 mM mannitol, 5 mM Na glutamate (or 20 mM for excess N), 250 µM CaCl₂.2H₂O, 37 µM FeCl₃.6H₂O, 48 µM H₃BO₃, 10 µM MnSO₄.H₂O, 1 µM NaMoO₄.2H₂O, 0.3 µM CoCl₂.6H₂O, 2 mM K₂HPO₄, and 4 µM biotin.

### Description of the drawings

- Fig. 1:: **Curdlan forms a capsule.** *Agrobacterium* sp. 31749 cells are grown for 7 days in Agrobacterium defined broth (ADB) and subjected to microscopic analyses. Cells are stained with aniline blue fluorophore and photographed with bright field (A, bar = 5 µm) and fluorescence (B, excitation at 330-385 nm, emission at 420-475 nm). A & B photographs are from the same field. (Note that this sample is prepared by firmly pressing the glass slide and the object cover together, resulting in the disruption of some cells from the cell aggregates.) Cells grown in ADB from strain 31749 (C) and 31749 *crdS* disruptant (D) are stained with uranyl acetate and viewed using electron microscopy (bar = 2 µm). Arrows in C indicate cells lacking a capsule. These strains are also subjected to scanning electron microscopy (E & F, bar represents 200 nm). Black arrow indicates capsule (E) or a cell surface lacking a capsule (F). The curdlan capsule appears as a dark cloud in C, and an irregular outer layer in E. The capsule is absent in a *crdS* mutant (D & F).
- Fig. 2.:: **Curdlan promotes cell aggregation.** (A) In liquid cultures of Agrobacterium defined broth (ADB), encapsulated cells form cell aggregates/flocs, while non-capsulated cells remain free and unattached. Bar represents 5 µm. (B) Liquid cultures are analysed using a particle counter with a 10-40 µm cutoff. The average size of the particles/flocs from the curdlan producing strain are clearly larger and shifted to right (compare 3 days and 6 days), while the particles from the *crdS* disruptant do not.
- Fig. 3:: Exemplary **genetic modifications of the *crdASC* locus.** Three versions of the suicide plasmid (A-C) with highlighted differences for ensuring genetic modification at the *crdASC* locus. A, the crdA::mVenus reporter construct (suicide plasmid 1, translational fusion) is inserted into the chromosome via homologous recombination (indicated with dashed line) using the PcrdA fragment fused to the gene for mVenus. The insertion of this construct provides a read-out of *crdA* expression without disrupting the *crdASC* gene cluster. B, suicide plasmid 2 includes an approximately 490 bp fragment from the *crdA* open reading frame for guiding homologous recombination. The fragment includes the ribosome binding site and the translation start of *crdA* (total length = 1458 bp). Insertion of this construct effectively knocks out (KO) transcription of the *crdASC* gene cluster, since this cluster now lacks a promoter, while the Pcrd controls only a fragment of *crdA.* C, the strong PphaP promoter sequence is fused to the fragment of *crdA.* Insertion of suicide plasmid 3 via homologous recombination ensures overexpression of the *crdASC* gene cluster due to the insertion of the strong PphaP promoter directly upstream of *crdA.* D, alignment of the DNA sequences of the *crd* and *phaP* promoters. The -35 and -10 promoter elements are indicated by a black line. The transcription start of PphaP (+1, bold) is identified based on RNA sequencing data from *Rhodobacter sphaeroides.*
SEQ ID 5 (alignment of DNA sequence of promoter Pcrd): 
GGAAAATTAGTTAATGCAATTTTTACTATGTTGCGGCGCACAAGAAC
TTCATTCACCATTTCAATAC,
SEQ ID 6 (Alignment of DNA sequence of promoter PphaP): 
CGAAAAAATGCTGCGGTGCAGAATGAGCGGTTGATTTTCTGCGGC
GCAGCATTTATATCCTTGTCAC.
- Fig. 4:: Example of **96-well plate assay revealing a temporal profile of the onset of curdlan production.** *Agrobacterium tumefaciens* C58 (C58 WT) and Agrobacterium sp. 31749 (31758 WT) are grown in a 96-well plate (8 wells for each strain). For the medium and other details of this setup, see section materials and methods, above. For the detection of curdlan, the medium contains 0.1 mg/mL aniline blue (0.01%). Additionally, a translational fusion between *crdA* and the gene for mVenus is inserted into the chromosome, allowing detection of *crdA* expression. Growth (A), fluorescence from aniline blue/curdlan (B) and fluorescence from mVenus (C) of the cultures is measured once every three hours for 60 hours using an automated Tecan reader setup. Black arrows at approximately 33 h indicate onset of curdlan production.
- Fig. 5:: **PphaP is more active than Pcrd.** Agrobacterium strains (A & B, C58, C & D 31749) incapable of curdlan production (via the pK-KO suicide vector) carrying the low copy plasmid pPHU231 containing the Pcrd::mVenus fusion (A & C) or the PphaP::mVenus fusion (B & D) are grown in a 96-well plate in various exemplary media. PY, peptone yeast, Glu, glucose, AE, apple extract (10%), MOPS, buffered minimal defined medium. Cultures are measured for mVenus fluorescence at the times indicated, and fluorescence per µL of bacterial culture is presented. Error bars indicate standard deviation from 8 well cultures.
- Fig. 6:: **PphaP increases curdlan production in minimal medium.** Bacteria are grown in a 96-well plate assay to reveal a temporal profile of the onset of curdlan production. Fluorescence from aniline blue/curdlan of the cultures is measured once every three hours for 60 hours using an automated Tecan reader setup. For the details of this setup, see the section materials and methods. Cultures include the C58-derivatives *crdA* knockout (KO), C58 wild type (WT) and C58 PphaP for (A) and likewise for the 31749-derivatives (B). Error bars indicate standard deviation from 8 wells.
- Fig. 7:: **PphaP increases curdlan production from apple extract (AE) and in defined medium with glucose.** (A), the modified strains of Agrobacteria are grown on 1% agar with 0.05% yeast extract and 1% tryptone containing 0.005% aniline blue with varying levels of AE or glucose as indicated. (B), bacteria are grown in ADB broth (4% glucose) Cultures (50 mL) are shaken (150 rpm) in 500 mL flasks at 30°C. Curdlan is harvested after 3 days (see materials and methods for details). Error bars indicate variation as standard deviation from three biological replicates. C58 does not produce any detectable curdlan.

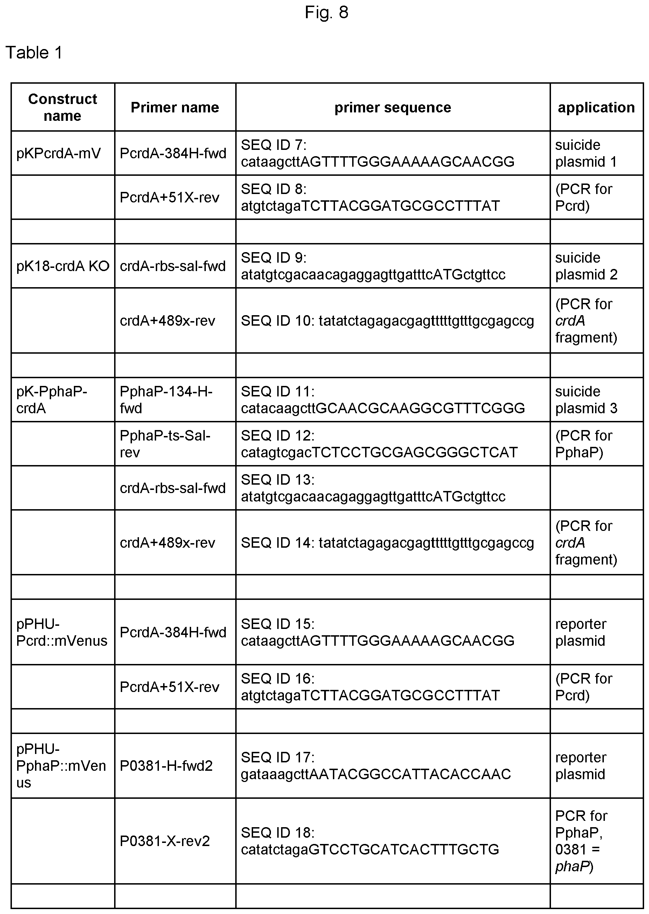

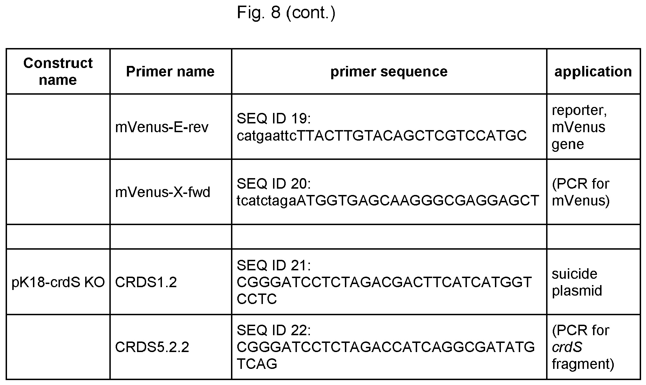

- Fig. 8:: Table 1, showing primers and plasmids exemplarily used within the scope of this invention.

## Claims

1. A transcription overriding cassette (TORC) composed for the expression of at least one gene of interest, **characterised in that** the transcription overriding cassette comprises the following parts i) and ii),
i) a first DNA sequence comprising a strong constitutive promoter DNA sequence having at least 50% nucleotide identity with the promoter DNA sequence according to SEQ ID 6;
ii) a second DNA sequence having at least 50% nucleotide identity with SEQ ID 23;
whereat
the DNA sequence according to i) is located directly before the DNA sequence according to ii) within the TORC and
the DNA sequences of part i) and part ii)
- are connected directly to each other within the complete sequence of the TORC or
- are separated from each other within the complete sequence of the TORC by DNA
sequences of DNA restriction cleavage sites.

2. A TORC according to claim 1, **characterized in that** the DNA sequence of the TORC has at least 50% nucleotide identity with SEQ ID 4.

3. A vector comprising a TORC according to any one of claims 1 or 2.

4. Usage of a TORC according to any one of claims 1 or 2 for the homologous or heterologous expression of at least one gene of interest in cells by transforming the TORC into these cells and cultivating them, whereat the promoter of the TORC is replacing the natural promoter of the at least one gene of interest.

5. Usage according to claim 4, **characterised in that** the at least one gene of interest is encoding at least one enzyme for the production of curdlan.

6. A recombinant cell of an Agrobacterium comprising the TORC of any one of claims 1 or 2 and the corresponding at least one gene of interest, whereat the at least one gene of interest is operatively linked, i. e. covalently bound, to the TORC and both, the TORC and the at least one gene of interest, are located within the Agrobacterial chromosome.

7. The Agrobacterial cell according to claim 6, **characterised in that** it is a cell of *Agrobacterium sp.* strain ATCC 31749 or *Agrobacterium tumefaciens* C58.

8. A method for transformation of a cell of an Agrobacterium with a TORC according to any of claims 1 or 2 by
a) providing of a TORC according to any of the
preceding claims 1 or 2;
b) transformation of a Agrobacterium with the TORC;
d) selection of the host cells that have been stably transformed with the TORC by using the respective selection agents;
e) isolating the transformed host cells.

9. A method of producing curdlan, **characterized in that** it comprises the following steps
a) providing cells of Agrobacteria according to claim 6 or according to claim 7 and a fermentation broth suitable for growing cells of Agrobacteria according to claim 6 or according to claim 7;
b) mixing the Agrobacteria and the fermentation broth according to step a) and performing a fermentation;
c) gathering the curdlan having been produced during step b) at least partially.

10. The method according to claim 9, **characterized in that** step c) comprises filtering the fermentation broth through a filter having a pore size which allows the separation of flocks of bacteria which have already produced curdlan from bacteria which have not yet produced curdlan, the pore size being within the range of 0.03 mm - 5.0 mm.

11. The method according to claim 9, **characterized in that** step c) comprises the performance of a sedimentation process upon the fermentation broth until the flocks of bacteria which have already produced curdlan are settled down and the bacteria which have not yet produced curdlan are still floating, the means for performing the sedimentation process being either centrifugation or subjecting the fermentation broth to undisturbed natural gravity.
